**Europäisches Patentamt**

**European Patent Office**

**Office européen des brevets**

(19)

(11) **EP 1 258 242 A1**

(12) **EUROPÄISCHE PATENTANMELDUNG**

(43) Veröffentlichungstag:
**20.11.2002 Patentblatt 2002/47**

(51) Int Cl.$^7$: **A61K 9/48**

(21) Anmeldenummer: **01111739.7**

(22) Anmeldetag: **15.05.2001**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(71) Anmelder: **Swiss Caps Rechte und Lizenzen AG
9533 Kirchberg (CH)**

(72) Erfinder:
• **Engel, Dieter Wolfgang
9524 Zuzwil (CH)**

• **Brocker, Erich, Dr.
9533 Kirchberg (CH)**
• **Ménard, Rico
9533 Kirchberg (CH)**

(74) Vertreter: **Wenger, René et al
Hepp, Wenger & Ryffel AG
Friedtalweg 5
9500 Wil (CH)**

(54) **Verfahren zum Herstellen von Formkörpern, insbesondere von Weichkapseln**

(57) Die Erfindung betrifft ein Verfahren zum Herstellen von Formkörpern, insbesondere von Weichkapseln ausgehend von einem Rohstoffgemisch aus wenigstens einem pflanzlichen Biopolymer in Pulver- oder Granulatform und wenigstens einem Weichmacher in flüssiger Form, insbesondere in Form eines Sirups. Nach dem Aufschmelzen des Rohstoffgemisches unter Zufuhr von Wärme und unter erhöhtem Druck zu einer thermoplastisch verarbeitbaren Masse wird ein Film ausgebildet. Der Film dient zur Herstellung von Formkörpern, insbesondere im Rotary-Die-Verfahren. Der Feuchtigkeitsgehalt aller Komponenten wird über sämtliche Prozessstufen derart gewählt bzw. gesteuert, dass die Restfeuchte der fertigen Formkörper nach dem Herstellen der Formkörper unter Vermeidung eines Trocknungsprozesses dem gewünschten Wert bei Lager- bzw. Gebrauchsbedingungen entspricht.

Fig.3

EP 1 258 242 A1

**Beschreibung**

Die Erfindung betrifft ein Verfahren zum Herstellen von Formkörpern, insbesondere von Weichkapseln, gemäss dem Oberbegriff von Anspruch 1.

Formkörper aus biologisch abbaubaren Materialien sind aus Gründen des Umweltschutzes schon seit längerem von ausserordentlichem Interesse. Als Folge der BSE-Problematik gewinnen insbesondere Kapseln mit einer Kapselhülle aus gelatinefreien Materialien für die Verabreichung pharmazeutisch wirksamer Substanzen an Bedeutung.

In einer Reihe von Publikationen wird die Herstellung von Steckkapseln aus Stärke beschrieben, wie z.B. in EP 118 240 und US 4,738,724. Die Steckkapseln werden als zweiteilige Hülle im Spritzgussverfahren vorfabriziert und, gegebenenfalls nach Zwischenlagerung, mit hochviskosen oder festen Wirksubstanzen gefüllt. Aufgrund von Undichtigkeiten der Steckverbindung eignen sich Steckkapseln nicht für niedrigviskose Flüssigkeiten. Zudem ist der Herstellungsprozess einer gefüllten Steckkapsel aufwendig und kostspielig, da die Arbeitsschritte Herstellen und Füllen der Kapselhülle getrennt voneinander vorgenommen werden.

Für flüssige, im weitesten Sinne pumpbare Kapselinhaltsstoffe haben sich Kapseln mit einer einteiligen Kapselhülle aus Gelatine durchgesetzt, die in kontinuierlichen, automatisierbaren Verfahren hergestellt werden können. Die Herstellung der Kapselhülle und das Füllen derselben geschieht dabei in einem einzigen Arbeitsschritt. In diesen kontinuierlichen, 1-Schritt-Verfahren werden Formteile gefertigt, aus denen die Kapselhülle während und nach dem Füllen durch Verschweissen der Aussenkanten der Formteile zusammengefügt werden. Die Formteilfertigung geschieht entweder mittels auseinander- und zusammengehender Formen, wie z.B. im Norton- , Banner und Schering-Prozess oder mittels rotierender Formwalzen, wie es z.B. im Rotary-Die-Prozess und im Accogel-Verfahren verwirklicht ist ("Die Kapsel" Fahrig/Hofer-Herausgeber, Stuttgart, 1983; Lachmann/Liebermann/Kanig, "The Theory and Practice of Industrial Pharmacy"; Third Edition, Philadelphia 1986). Das Füllen erfolgt mit Hilfe von Dosierpumpen, die eine definierte Menge Wirksubstanz während des Ausstanzens und Verschweissens der Formteile zur Bildung einer einteiligen Kapselhülle abgeben. Das Verschweissen, d.h. die Ausbildung der Nähte erfolgt generell durch Druck und Wärme. Die Herstellkosten sind gegenüber der Herstellung von zweiteiligen Steckkapseln erheblich reduziert.

US 5,342,626 beschreibt die Herstellung von Kapseln im Rotary-Die-Prozess, wobei das Kapselhüllmaterial aus Carrageenan, Mannan-Gums, wie z.B. Galacto- und Glucomannanen, Gelan bzw. Mischungen untereinander besteht. Diese makromolekularen pflanzlichen Biopolymere sind jedoch aus Kostenüberlegungen inakzeptabel, da die Rohstoffe zu teuer sind.

Der Herstellungsprozess für einteilige Kapseln stellt an das Kapselhüllmaterial eine Reihe von Anforderungen. Eine der Hauptvoraussetzungen ist die Fähigkeit des Kapselhüllmaterials hochelastische "endlose" Bänder mit einer ausreichenden Festigkeit auszubilden. Die Kapselhülle muss sich bei Bedarf im Magendarmtrakt rasch lösen, um die Wirksubstanzen freisetzen zu können. Das Kapselhüllmaterial muss verschweissbar sein. Die Moleküle des die Formteile bildenden Materials, insbesondere die Makromoleküle des Polymeren, sollten sich an der Nahtstelle idealerweise durchdringen, um eine ausreichende Stabilität der Nahtstelle zu gewährleisten. Gelatine erfüllt all diese Bedingungen in nahezu idealer Weise und konnte als Material für einteilige Kapselhüllen bislang nicht ersetzt werden.

Unter Verfügbarkeits- und Kostenkriterien ist Stärke auch für die Herstellung einteiliger Kapselhüllen ein wünschenswertes Ausgangsmaterial.

Die Herstellung von Stärkefilmen wurde schon mehrfach beschrieben, die Kombination von Eigenschaften, die ein solcher Stärkefilm zur Herstellung einteiliger Kapselhüllen aufweisen muss, wurde bis anhin jedoch nicht offenbart.

EP 474 705 beschreibt ein Verfahren zur Herstellung von Stärkeformkörpern durch Extrusion einer Stärkeschmelze. Die Stärkeschmelze enthält Stärke mit einem Amylosegehalt über 50 % und Zuschlagstoffen. Aus der Schmelze wird vor, während und/oder nach dem Extrudieren das Wasser durch Anlegen von Unterdruck entfernt. Die aus diesem Material extrudierten Folien weisen eine Bruchdehnung zwischen 80 und 200% auf. Hochamylosehaltige Stärken sind als Kapselhüllmaterial nicht geeignet, da die Tendenz der Amyloseketten zur Retrogradation einem schnellen Auflösen der Kapselhülle entgegensteht.

EP 0 397 819 offenbart ein Verfahren zum Herstellen thermoplastisch verarbeitbarer Stärke, wobei der kristalline Anteil in der Stärke unter 5% liegt. Das Verfahren besteht im Mischen nativer Stärke mit mindestens 10 Gew. % eines Zuschlagstoffes, welcher einen Löslichkeitsparameter von mindestens 30,7 $(MPa)^{1/2}$ besitzt. Die Mischung wird unter Wärmezufuhr in einem Temperaturbereich zwischen 120°C und 220°C in eine Schmelze überführt. Der Wassergehalt der Stärke wird bereits in der Schmelze auf unter 5% reduziert. Die Molmasse der eingesetzten Stärke ist vor der Überführung in den thermoplastischen Zustand grösser als 1 000 000 Dalton, bevorzugt zwischen 3 000 000 Dalton und 10 000000 Dalton. Dieses Verfahren liefert zwar eine thermoplastische Stärke mit guter Verarbeitbarkeit zu Formkörpern, welche eine ausreichende Festigkeit aufweisen, die Bruchdehnung der mit dieser thermoplastischen Stärke hergestellten Formkörper erreicht jedoch nur Werte zwischen 40 und 55%. Die Elastizität der Stärkefilme ist damit für die Herstellung einteiliger Kapselhüllen in kontinuierlichen Verfahren zu gering und führt zu einem Reissen der Formteile bei der Herstellung bzw. zu Rissen in der fertigen Kapsel. Auch zeigt der Stärkefilm nicht die Verschweissbarkeit bzw. Nahtfestigkeit, die den Qualitätsanforderungen, die an einteilige Kapselhüllen gestellt werden, genügt.

EP 304 401 beschreibt ebenfalls ein Verfahren zur Herstellung geformter Gegenstände aus Stärke. Die dazu notwendige thermoplastische Stärkeschmelze wird aus einer vorbehandelten Stärke hergestellt. Die Destrukturierung (Zerstörung der kristallinen Bereiche) der nativen Stärke und die anschliessende Homogenisierung (Überführen in den thermoplastischen Zustand) findet jeweils bei Temperaturen zwischen 120°C und 190°C in einem geschlossenen Gefäss mit einem Wassergehalt zwischen 10 und 20% statt. Die Bruchdehnung der nach diesem Verfahren hergestellten Stärkefilme ist für die Produktion einteiliger Kapselhüllen nicht ausreichend. Die Stärkefilme zeigen darüber hinaus auch eine unzureichende Verschweissbarkeit und Nahtfestigkeit.

EP 0 542 155 offenbart biologisch abbaubare Formmassen, die sich unter anderem zur Filmherstellung eignen. Die Formmassen enthalten neben thermoplastisch verarbeitbaren Stärke Zellulosederivate. Die Bruchdehnung übersteigt den Wert von 85 % jedoch nicht, was für die Herstellung einteiliger Kapselhüllen in kontinuierlichen Verfahren nicht ausreicht. Die Verschweissbarkeit der Filme ist unbefriedigend. Viele der in EP 542 155 offenbarten Polymerblends enthalten Substanzen, die für pharmazeutische Anwendungen und für Nahrungsmittel nicht zugelassen sind.

WO 97/35537 offenbart mittels rotierender Formwalzen hergestellte einteilige Kapseln, die gelierte Stärke enthalten. Das teilweise Anlösen der Filmoberfläche hat sich in der Herstellung einteiliger Kapseln in Bezug auf Transport- und Druckstabilität als nachteilig erwiesen (beim Herausdrücken der Kapseln aus den Blisterverpackungen). Die Kapselhüllen werden dadurch im Bereich der Nahtstelle zu weich und zu flexibel.

Ein weiteres Problem, das gemäss vorliegendem Stand der Technik nicht gelöst ist und den Herstellungsprozess von Kapseln zum Beispiel im Rotary-Die-Verfahren unnötig verlängert und auch verteuert, ist der jeweilige Feuchtigkeits- beziehungsweise Wassergehalt des zu den Kapseln zu verarbeitenden Materials und auch der Kapsel als Endprodukt des Verfahrens. Ist der Feuchtigkeitsgehalt des Ausgangsmaterials zu niedrig, wird das in einem ersten Verarbeitungsschritt hergestellte Band hart und brüchig und kann im folgenden Rotary-Die-Verfahren nicht zu Kapseln geformt werden. Enthält das Material zu viel Feuchtigkeit, ist das hergestellte Band zwar elastisch, jedoch klebrig. Um aus einem solchen Band eine Kapsel herstellen zu können, muss der Film getrocknet werden.

Eine weiteres, vom Stand der Technik bislang nicht gelöstes Problem ist der Feuchtigkeitsgehalt der Kapsel nach ihrer Fertigstellung. Auch hier zeigt sich, dass Kapseln aus zu trockenem Material spröde und rissig werden; ist der Feuchtigkeitsgehalt zu hoch, kleben sie und lassen sich beispielsweise schlecht aus der Verpackung lösen.

Die Aufgabe der vorliegenden Erfindung ist die Vermeidung der Nachteile des Standes der Technik. Insbesondere besteht die Aufgabe der vorliegenden Erfindung darin, ein Verfahren der eingangs genannten Art bereitzustellen, bei dem aufwendige Trocknungsmassnahmen während oder nach der Fertigstellung der Formkörper entfallen, so dass die Restfeuchte eines Zwischenprodukts bzw. des Endprodukts unmittelbar dem angestrebten Wert bei normalen Gebrauchsbedingungen mit weniger als 50 % relativer Luftfeuchtigkeit und unterhalb von 30° Celsius entspricht.

Eine weitere Aufgabe besteht darin, Stärkekapseln mit einteiliger Kapselhülle bereitzustellen, welche nach einer Lagerdauer von wenigstens einem Jahr weder Undichtigkeiten zeigen, noch Veränderungen der Auflösungsgeschwindigkeit der Kapselhülle.

Eine anderer Aspekt der vorliegenden Erfindung besteht darin, dass die Kapseln ihre physikalischen Eigenschaften auch über längere Zeiträume hin bewahren.

Diese Aufgaben werden gelöst mit einem Verfahren, das die Merkmale im Anspruch 1 aufweist. Das Verfahren kann dabei insbesondere auch noch folgende Schritte umfassen:

- Überführung einer Mischung enthaltend mindestens eine Stärke, Wasser, und mindestens einen organischen Weichmacher, unter Erhitzen und Kneten in eine thermoplastisch verarbeitbare, vorzugsweise homogenisierte, Masse in einer ersten Verarbeitungseinrichtung;
- gegebenenfalls Herstellen eines lagerfähigen Zwischenproduktes, insbesondere eines Granulates nach Abkühlen der im ersten Schritt erhaltenen Masse und nachfolgendes Überführen des Zwischenproduktes in eine thermoplastisch verarbeitbare Masse in einer zweiten Verarbeitungseinrichtung;
- Herstellen wenigstens eines Materialsstranges, insbesondere eines extrudierten Films, am Ausgang der ersten oder gegebenenfalls zweiten Verarbeitungseinrichtung,
- Umformen des Materialstranges zu einem Formkörper in einem kontinuierlichen oder intermittierenden Formverfahren;

wobei die ersten Schritte derart durchgeführt werden, dass beim Formen der Formkörper der Staudinger-Index [η] der Stärke in der den Materialstrang bildenden Masse einen Wert von nicht weniger als 40 ml/g, bevorzugt mindestens 50 ml/g und noch bevorzugter mindestens 80 ml/g aufweist. Noch bessere Eigenschaften werden erhalten, wenn der Staudinger-Index der Stärke einen Wert von grösser oder gleich 100 ml/g hat. Die vorteilhaftesten Eigenschaften werden erhalten mit einem Wert des Staudinger-Index der Stärke von grösser oder gleich 130 ml/g. Der Staudinger-Index darf einen maximalen Wert von 1000 ml/g nicht übersteigen. In einer vorteilhaften Ausführungsform übersteigt der Staudinger-Index 700 ml/g und noch bevorzugter 300 ml/g nicht.

Die im ersten Schritt eingesetzte Mischung enthält die Stärke vorzugsweise in einem Gewichtsbereich von 45 bis 80 Gew.% bezogen auf das Gesamtgewicht der Mischung.

Die Begriffe "thermoplastisch verarbeitbar", "Schmelze", "Sirup" und "amorph" werden definiert nach Römpp Chemie Lexikon, Hrsg: J. Falbe, M. Regitz, 9. Auflage, 1992, Georg Thieme Verlag, Stuttgart.

Unter dem Begriff Stärke sollen native Stärken, sowie physikalisch und/oder chemisch modifizierte Stärken verstanden werden. Für die in Schritt a) des erfindungsgemässen Verfahrens eingesetzte Mischung sind alle Stärken, unabhängig von der Pflanze aus der sie gewonnen werden, geeignet. In einer bevorzugten Ausführungsform handelt es sich um Stärke, deren Amylopektingehalt über 50% bezogen auf das Gesamtgewicht der wasserfreien Stärke liegt. Als für das Verfahren bevorzugt haben sich physikalisch und/oder chemisch modifizierte Kartoffelstärken erwiesen.

Für die vorliegende Erfindung sind jedoch im weitesten Sinne alle Polyglucane, d.h. 1.4 und/oder 1.6 Poly-$\alpha$-D-glucane und/oder Abmischungen zwischen diesen geeignet.

In einer bevorzugten Ausführungsform ist die Stärke eine hydroxypropylierte Stärke. Der Substitutionsgrad (DS = degree of substitution) ist dabei im Bereich von 0.01 bis 0.5, vorzugsweise im Bereich von 0.05 bis 0.25 und noch bevorzugter im Bereich von 0.1 bis 0.15. Insbesondere handelt es sich um hydroxypropylierte Kartoffelstärke.

In einer weiteren bevorzugten Ausführungsform handelt es sich bei der Stärke um vorverkleisterte Stärke. Oberhalb einer für jede Stärkeart typischen Temperatur tritt in wässrigen Stärkesuspensionen nach Erreichen eines Höchstquellungsgrades "Lösung" der Stärkekörner ein, d.h. irreversible Desintegration der Stärkekörner. Dieser Vorgang wird auch als "Verkleisterung" bezeichnet. Die Verkleisterung, d.h. die irreversible Quellung der Stärkekörner bei höherer Temperatur bis zum 40-fachen des ursprünglichen Volumens beruht auf einer allmählichen Wasseraufnahme und Lösung von Wasserstoffbrücken-Bindungen, die eine weitere Hydratation bis zur völligen Desintegration des Stärkekorn-Gefüges ermöglicht.

Die Überführung der Stärke enthaltenden Mischung in den thermoplastischen, vorzugsweise homogenisierten Zustand, ebenso wie die danach folgenden Verarbeitungsschritte, müssen unter Bedingungen erfolgen, die einen unkontrollierten Abbau der Amylose- und Amylopektinmoleküle zu kurzen Bruchstücken verhindern.

Es muss das Zusammenwirken aller Verarbeitungsparameter wie z.B. Temperatur, Druck, Verweilzeit und Knetleistung, während der verschiedenen Schritte berücksichtigt werden, um einen weitgehenden Abbau der Stärkemoleküle zu verhindern. So kann z.B. auch bei relativ hohen Temperaturen ein weitgehender Abbau der Stärkemoleküle vermieden werden, wenn die Verweilzeiten der Stärke enthaltenden Masse bei diesen Temperaturen klein gehalten wird.

In einer bevorzugten Ausführungsform übersteigt die Temperatur der Masse in der ersten und gegebenenfalls zweiten Verarbeitungseinrichtung, sowie beim Herstellen des Materialstranges 160°C , bevorzugt 140°C, noch bevorzugter 120°C und am vorteilhaftesten 90°C nicht. Bei 160°C sollte insbesondere auch der Aufschlussvorgang in Schritt a) in weniger als 5 Minuten, bevorzugt weniger als 3 Minuten abgeschlossen sein.

In einer weiteren bevorzugten Ausführungsform überschreitet die in die Masse durch das Kneten eingebrachte Energie zur Erzeugung einer thermoplastisch verarbeitbaren homogenisierten Masse in Schritt a) bis c) 0,3 kWh/kg, bevorzugt 0,2 kWh/kg und noch bevorzugter 0,175 kWh/kg nicht.

Die Überführung in den thermoplastisch verarbeitbaren Zustand bewirkt eine irreversible Quellung der Stärkekörner, was eine Voraussetzung dafür ist, dass die Masse in den homogenen Zustand überführt werden kann bzw. auch nach dem Abkühlen homogenisiert vorliegt. Durch die Schritte a) bis c) wird weiterhin eine Masse erzeugt, in der auch im wesentlichen keine kristallinen Bereiche in der Stärke mehr vorhanden sind. Kristalline Bereiche führen im Materialstrang zu Stippenbildung, d.h. zu Inhomogenitäten, die sich besonders dann nachteilig auswirken, wenn der Materialstrang in Schritt c) ein extrudierter Film ist. Mit "im wesentlichen keine kristallinen Bereiche" soll gemeint sein, dass diese soweit zerstört sind, dass eine Beeinträchtigung der bezüglich der Umformung relevanten physikalischen Parameter des extrudierten Materials nicht auf das Vorhandensein kristalliner Bereiche zurückgeführt werden kann.

Unter dem Begriff "homogene Masse/Material" bzw. "homogenisierte Masse/Material" soll somit ein Material oder eine Masse verstanden werden, die an jeder Stelle im Material die im wesentlichen gleichen physikalische Eigenschaften (Parameter) aufweist. Zu geringfügigen Abweichungen kann es an den jeweiligen Material- oder Formteiloberflächen durch Aufnahme von Luftfeuchtigkeit kommen. Homogen bzw. homogenisiert liegt die Masse dann vor, wenn unter dem Mikroskop die Anzahl der noch sichtbaren Stärkekörner im Schnitt unter einem Prozent liegt. Dazu wird die Masse im thermoplastischen Zustand abgekühlt, in dünne Scheiben geschnitten und unter dem Lichtmikroskop analysiert.

Eine homogenisierte Masse/Material wird erhalten unter Überführen der Mischung in den erweichten oder sogar flüssigen Zustand, der in einen thermoplastisch verarbeitbaren Zustand resultiert. Der Grossteil der die Mischung ausmachenden Komponenten (Stärke, organischer Weichmacher, Gleit- und Formtrennmittel) kann dabei im geschmolzenen Zustand vorliegen und bei genügend langer Stand- und/oder Misch (Knet-)dauer wird die Masse an jeder Stelle der Schmelze die im wesentlichen gleichen Eigenschaften oder chemische Zusammensetzung haben (homogene Masse). Dieser homogene Zustand wird auch bei und nach Abkühlen des thermoplastischen Zustands beibehalten. Es treten keine Entmischungsvorgänge ein. Dies sorgt für gleichmässige mechanische Eigenschaften des Formkörpers bei Raumtemperatur.

Der Staudinger-Index [η] oder auch Grenzviskosität steht innerhalb einer polymerhomologen Reihe mit der Molmasse, dem Gewichtsmittel der Molekulargewichtsverteilung, in folgendem Zusammenhang

$$[\eta] = K \times M^{\alpha.}.$$

wobei $\alpha$ ein von der Molekül-Gestalt abhängiger Exponent und der K-Wert eine von der gelösten Substanz und vom Lösungsmittel abhängige Konstante ist. Der Staudinger-Index ist innerhalb der polymerhomologen Reihe um so grösser, je grösser das Molekulargewicht des Polymeren bei ansonsten unveränderten Parametern ist. Eine Ermittlung der absoluten Molekulargewichte kann die Messung des Staudinger-Index nicht leisten.

Die Bestimmung von absoluten Molekularmassen bei Stärken ist bekanntermassen überaus schwierig und das Ergebnis ist sehr stark abhängig von der verwendeten Messmethode. Dies gilt um so mehr, je verzweigter die Moleküle sind. Die Ergebnisse der absoluten Molekularmassenbestimmung ist deshalb auch für Amylopektin bzw. amylopektinhaltige Stärke mit einem hohen Unsicherheitsgrad versehen. Da die absolute Molekularmassenbestimmung darüber hinaus sehr teuer ist, liefert die Messung des Staudinger-Index schnellere, verlässlichere und dem Zweck angepasstere Werte.

Ohne eine erschöpfende Erklärung zu liefern, wird vermutet, dass sich in erster Linie der Polymerisationsgrad der Amylopektinmoleküle in der eingesetzten Stärke für die Elastizität und damit für eine möglichst hohe Bruchdehnung des in Schritt d) erzeugten Materialstranges verantwortlich zeigt. Eine hohe Bruchdehnung ist insbesondere für einen bahnförmigen Film, der im Rotary-Die-Verfahren zu einer Weichkapsel geformt werden soll, von grosser Bedeutung.

Es besteht die Vorstellung, dass zusätzlich zur inhärenten Elastizität der Stärkegele, die bei genügendem Polymerisationsgrad der sie konstituierenden Amylopektinmoleküle sowieso gegeben ist, auch eine Art "Stärke-Netzwerk" entstehen kann, das durch Verschlingung und Verhakung der Amylopektinmoleküle aufgebaut wird, und durch Verzweigungen des Moleküls unterstützt wird. Aber auch Amylosemoleküle können bei genügend hohem Polymerisationsgrad, an diesem "Stärke-Netzwerk" partizipieren. Auch die chemische Substitution der Stärkehydroxlygruppen unter Ether-, Ester-, Vinyl- und Acetalbildung können vorteilhaft sein, da sie die Ausbildung von Stärkenetzwerken fördern.

Der im letzten Schritt erhaltene Formkörper weist den im wesentlichen gleichen Polymerisationsgrad der Stärke auf, den die vorhergehenden Schritte bewirkt haben.

Das Vorhandensein dieser Netzwerke und möglicherweise auch das Vorhandensein von analytisch nicht nachweisbaren und auch in Form von Stippenbildung nicht sichtbaren Nanokristallen (analog Weich-PVC) ist anscheinend für das Auftreten eines Gummiplateaus verantwortlich. Das Young'sche Elastizitätsmodul E amorpher, nicht vernetzter Polymere und insbesondere von linearen Polymeren, fällt normalerweise nach dem Durchlaufen des Bereichs der Glasübergangstemperatur mit steigender Temperatur nahezu linear bis auf 0°C ab. Die Polymere verhalten sich bei genügend hoher Temperatur wie eine Flüssigkeit. Das Charakteristikum eines Gummiplateaus ist demgegenüber, dass die mechanischen Eigenschaften wie das Young'sche Elastizitätsmodul E, die Bruchdehnung $\varepsilon_B$, die maximale Festigkeit $\sigma_m$, etc. über eine längere Temperaturspanne annähernd konstant und nahezu unabhängig von der Temperatur bleiben. Ein Gummiplateau ist normalerweise nur bei vernetzten (chemischen Vernetzung) Polymeren zu beobachten (vgl. Intruduction to Polymers, Hrsg. R. J. Young, P. A. Lovell, Chapman und Hall, London, 2. Auflage 1991, Seite 344/345). Unerwarteterweise weisen die Massen der vorliegenden Erfindung trotz Ausbleiben einer dreidimensionalen chemischen Vernetzung ein Gummiplateau auf.

Vor diesem Hintergrund können auch die vorteilhaften Eigenschaften eines 1.4 und 1.6 Polyglukans, das mit kurzen linearen Ketten von 1.4 Polyglukanen kokristallisiert sind, verstanden werden. Durch die Kokristalisierung entstehen zum einen weitere Verzweigungen, die sich auf das Ausbilden eines Netzwerkes positiv auswirken und zum anderen nicht sichtbare Nanokristalline-Bereiche. Bevorzugt werden als 1.4 und 1.6 Polyglukanen Amylopektine eingesetzt.

Die erfindungsgemässen und mit dem erfindungsgemässen Herstellungsverfahren erhaltenen Massen zeigen im Temperaturbereich von etwa 20°C bis ca. 80°C mechanische Eigenschaften, wie z. B. $\varepsilon_B$, $\sigma_m$, E, die im Wesentlichen unabhängig von der Temperatur sind. Das Gummiplateau ist für das Umformen und Füllen der Filme in gefüllte Formkörper von ausschlaggebender Bedeutung. So weist das Young'sche Elastizitätsmodul E des erfindungsgemässen Stärke enthaltenden Films im Augenblick des Umformens und Befüllens im Rotary-Die-Prozess maximal 2 MPa, bevorzugt maximal 1 MPa auf. Mit anderen Worten, der Film darf dem Fülldruck des Füllmaterials, der letztendlich die Ausformung der Kapselhülle im Rotary-Die-Prozess bewirkt, bei dem durch die Maschine gegebenen Auflagedruck des Füllkeils nicht einen solchen Widerstand entgegensetzen, dass das Füllmaterial zwischen Film und Füllkeil herausläuft. Es ist eben die Temperaturunabhängigkeit von $\varepsilon_B$ und $\sigma_m$ zwischen 40°C und 90°C, die die Verarbeitbarkeit der aus diesen Massen hergestellten Filme zu Weichkapseln im Rotary-Die-Verfahren ermöglicht.

Der Umformungsvorgang des Materialstranges zu einem Formkörper, insbesondere die Umformung eines extrudierten Films in eine einteilige Weichkapsel mit den in der Technik bekannten Verfahren, erfordert Bruchdehnungen des Materialstranges, insbesondere des Films von mindestens 100% im Bereich von 40°C bis 90°C bevorzugt von 60°C bis 80°C. In einer bevorzugten Ausführungsform ist die Bruchdehnung des Materialstranges, insbesondere Films, mindestens 160% und noch bevorzugter mindestens 240%.

Die Festigkeit $\sigma_m$ des Materialstranges, insbesondere des daraus hergestellten Formkörpers muss bei 25°C und 60% relativer Luftfeuchtigkeit wenigstens 2 MPa betragen. In einer bevorzugten Ausführungsform ist $\sigma_m$ grösser oder gleich 3.5 MPa und noch bevorzugter grösser oder gleich 5 MPa. Dieser Wert gewährleistet bei Raumtemperatur eine genügende Stabilität der Kapselhülle (Verpackung, Lagerung, Transportsicherheit und Gebrauch).

Das Befüllen erfolgt jedoch bei erhöhter Temperaturen des Filmes, die einen Fülldruck von nicht mehr als 2 MPa notwendig macht. Dies ist mit einem Young'schen Elastizitätsmodul E von kleiner oder gleich 2 MPa bei Verkapselungstemperatur (40°C bis 90°C) bei der vorliegenden Masse gegeben. Dies wurde bei den Ausführungen zum Gummiplateau bereits erläutert.

Der Gesamtweichmachergehalt der in Schritt a) eingesetzten Mischung beträgt mindestens 12 Gew.% bezogen auf das Gewicht der wasserfreien Stärke. In einer bevorzugten Ausführungsform ist der Gehalt des Weichmachers in einem Bereich von 30 Gew.% bis 60 Gew.% und noch bevorzugter in einem Bereich von 38 Gew.% bis 55 Gew.%.

Durch die erfindungsgemässe Prozessführung gelingt der weitgehende Ausschluss stark abgebauter Oligomere der Stärke. Dies erlaubt hohe Gesamtmengen an Weichmachern in die Masse einzuarbeiten. Die bei den Homogenisierungsmethoden des bisherigen Standes der Technik entstehenden Oligomere enfalten ebenfalls weichmachende Wirkung und die Einarbeitung grosser Mengen an Weichmacher wäre nicht möglich.

Bevorzugt werden solche Weichmacher eingesetzt, die einen Löslichkeitsparameter von gleich oder > 16,3 $(MPa)^{1/2}$ aufweisen. Die organischen Weichmacher werden ausgewählt aus der Gruppe bestehend aus Polyalkoholen, organischen Säuren, Aminen, Säureamiden und Sulfoxiden. Bevorzugt sind Polyalkohole. Aber auch Wasser wirkt als Weichmacher und bildet damit einen Teil des Gesamtweichmachergehalts. Der Wassergehalt der in Schritt a) eingesetzten Mischung liegt in einem Bereich von 6 bis 30 Gew.% bezogen auf die Gesamtmischung.

Der Wasseranteil der in Schritt a) eingesetzten Mischung kann im erfindungsgemässen Verfahren in Schritt b) oder c) gezielt verändert werden. Die physikalischen Parameter, die vom Wassergehalt abhängen, können so Veränderungen unterworfen werden.

Der in Schritt a) eingesetzten Mischung kann je nach erforderlichen Eigenschaften des in e) resultierenden Formkörpers noch mindestens ein Zuschlagstoff in einem Gewichtsbereich von 3,5 Gew.% bis 15 Gew.%, bevorzugt von 5 Gew.% bis 8 Gew.% bezogen auf das Gesamtgewicht der Mischung zugesetzt werden. Die Zuschlagstoffe werden ausgewählt aus der Gruppe bestehend aus Carbonaten und Hydrogencarbonaten der Alkali- und Erdalkaliionen, weitere Zerfallshilfen, Füllstoffe, Farbstoffe, Antioxidantien, physikalisch und/oder chemisch modifizierte Biopolymere insbesondere Polysaccharide und pflanzliche Polypeptide.

Opazität der homogenisierten Masse wird z.B. bevorzugt mit dem Zusatz von Titaniumdioxid als Füllstoff erreicht.

Als Zerfallshilfe, für einen schnellen Zerfall der Kapselhülle werden bevorzugt Calciumcarbonat und Amylasen zugesetzt.

Die Gruppe der physikalisch und/oder chemisch modifizierten Biopolymere umfasst unter anderen Cellulose, insbesondere teilhydroxypropylierte Cellulose, Alginate, Pektine, Agar, Carrageenan (lambda-, iota- oder kappa-Carrageenan), Galactomannane (Guarund Johannisbrotkernmehl), Xanthanlösung, Tamarinde, Tragantgummi, Karaya-Gummi, Chitosan, Glucomannane, Casein, Dextrine, Cyklodextrine, Pullulan und Arabinogalaktan.

In einer bevorzugten Ausführungsform enthält die in Schritt a) eingesetzte Mischung zusätzlich ein internes Gleit- und Formtrennmittel, welches ausgewählt ist aus der Gruppe bestehend aus Lecithinen, Mono-, Di- oder Triglyceriden von Speisefettsäuren, Polyglycerinester der Speisefettsäuren, Polyethylenglycolester der Speisefettsäuren, Zuckerester der Speisefettsäuren und Speisefettsäuren.

Das Gleit- und Formtrennmittel ist in der Mischung bevorzugt in einem Bereich von 0 bis 4 Gew.% bezogen auf das Gesamtgewicht der Mischung enthalten. Vorzugsweise wird es der Mischung in 0,5 bis 2 Gew.% und noch bevorzugter in 0,8 bis 1,5 Gew.% zugesetzt.

Vorteilhaft ist das Gleit- und Formtrennmittel ausgewählt aus der Gruppe bestehend aus Glycerinmonostearat und Lecithin.

Unter Speisefettsäuren werden die als Säurekomponenten der Triglyceriden natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine grosse Gruppe der Fettsäuren sind gesättigte Fettsäuren.

Die extrudierten Bänder werden nun entweder direkt weiterverarbeitet oder gegebenenfalls zur Lagerung unter Verwendung von Kunststofffolien als Zwischenschicht auf Rollen aufgewickelt. Als geeignetstes Folienmaterial hat sich dabei Polyethylen erwiesen.

Der mittels des erfindungsgemässen Verfahrens erhaltene Stärkefilm kann insbesondere für Herstellung von Weichkapseln auf allen in der Technik bekannten Anlagen zur Herstellung einteiliger Kapseln verarbeitet werden. Als besonders geeignet haben sich kontinuierliche Anlagen und insbesondere der Rotary-Die-Prozess erwiesen. Die Kapselwand wird dabei aus zwei vorab aus einem Stärkefilm herausgestanzten Formteilhälften unter Wärmewirkung bevorzugt grösser oder gleich 50°C verschweisst. Zwei "endlose Stärkefilme" werden durch zwei benachbarte, in gegenläufigem Sinn rotierenden Rollen oder Walzen mit Aussparungen geführt. Während der Stärkefilm durch den Fülldruck der Füllmasse in die Aussparung gepresst und somit die Kapselhälften geformt werden, wird die pump- und spritzbare Kapselfüllung mittels eines Ventils exakt dosiert und über einen Füllkeil in den Einzugswinkel der Formwalzen eingebracht. Die Form und Grösse der Kapsel ist somit abhängig von den geometrischen Abmessungen der Aussparungen in den Walzen und dem eindosierten Füllvolumen.

Konsequenterweise soll unter dem Begriff Kapsel deshalb nicht nur die typischen Kapselformen verstanden werden, sondern auch jede andere mögliche Form von "Hüllen", wie z.B. Kugeln, Kissen und Figuren. Bis heute existieren zahlreiche Weiterentwicklungen und Abweichungen von diesem grundlegenden Prinzip.

Die mittels des erfindungsgemässen Stärkefilms hergestellten einteiligen Kapselhüllen können zusätzlich beschichtet werden, z.B. um die Freisetzung von Wirksubstanzen zu verzögern, zu ästhetischen Zwecken, wie einer Glanz- oder Farbgebung, aber auch um den Feuchtigkeitsgehalt des Kapselmaterials konstant zu halten, damit diese nicht brüchig oder klebrig werden und ihre physikalischen Eigenschaften bewahren. Derartige Beschichtungen können aus Wachsen und/oder Lipiden bestehen und werden ausgewählt aus der Gruppe bestehend aus Bienenwachs (E901), Karnaubawachs (E903), Candelillawachs (E902), Beeren-Wachs, Montanglycol-Wachs (E912), Polyethylenglykol-Wachsoxidate (E914), Shellac (E904), Mono-, Di- und Triglyceridevon Speisefettsäuren (E471, Zuckerester von Speisefettsäuren (E476) und Dimethylpolisiloxan (E900).

Die Coextrusion, Beschichtung und das Laminieren des erfindungsgemässen Stärkefilms mit Materialien, deren filmbildende Eigenschaft auf synthetischen und/oder natürlichen Polymeren beruht, verschafft zusätzlich Möglichkeiten bestimmte Eigenschaften der Kapselhülle durch eine Mehrschichtfolie zu gestalten.

Insbesondere lässt sich durch den mehrschichtigen Aufbau eine Stärkefolie herstellen, die auf der Innenseite eine gut schweissbare Beschichtung aufweist, während die Aussenseite derart beschichtet wird, dass eine Retardwirkung des Zerfalls der Kapsel eintritt.

Teil der vorliegenden Erfindung ist weiterhin eine homogenisierte, Stärke enthaltende Masse, welche mindestens eine im wesentlichen amorphe Stärke enthält, die vorzugsweise in einem Gewichtsbereich von 45 bis 80 Gew. % bezogen auf das Gesamtgewicht der Masse vorliegt, die Masse enthält weiterhin Wasser, mindestens einen organischen Weichmacher in einem Anteil von mindestens 12 Gew.% bezogen auf das Gewicht der wasserfreien Stärke, wobei der Staudinger-Index der Stärke in der homogenisierten Masse mindestens 40 ml/g beträgt.

Bevorzugt ist ein Staudinger-Index der Stärke von mindestens 50ml/g, noch bevorzugter mindestens 80 ml/g. Besonders bevorzugt ist ein Staudinger-Index der Stärke von grösser oder gleich 100 ml/g. Noch bessere Eigenschaften werden erhalten mit einem Staudinger-Index der Stärke von grösser oder gleich 130 ml/g . Der Staudinger Index der Stärke darf 1000 ml/g, bevorzugt 700 ml/g und noch bevorzugter 300 ml/g nicht überschreiten.

Vorteilhaft wird eine Stärke mit einem Amylopektingehalt von grösser oder gleich 50 Gew.% bezogen auf das Gewicht der wasserfreien Stärke eingesetzt.

Der Gehalt an organischen Weichmachern liegt vorteilhaft im Bereich von 30 Gew.% bis 60 Gew.%, bevorzugt in einem Bereich von 38 Gew.% bis 55 Gew.% und noch bevorzugter in einem Bereich von 40 bis 50 Gew.% bezogen auf das Gesamtgewicht der Masse. Als Weichmacher können beipielsweise Glycerin, Sirup aus hydrierter, teilhydrolisierter Stärke, Abbauprodukte, enthaltend Oligosaccharide und Monosaccharide, Sorbitol, Maltitol, Mannitol, Erythritol, Xylitol, Spuren reduzierter Zucker und Gemische davon verwendet werden. In einer besonderen Ausführungsform sind wenigstens 1 Gewichtsprozent mehr eines von hydrolisierter und hydrierter Stärke stammenden Polyols, als von anderen Weichmachern vorhanden.

Per definitionem muss ein Sirup mindestens 70% w/w Dextrose-Äquivalente enthalten (Zuckerarten-Verordnung der Bundesrepublik Deutschland vom 24.4.1993 (BGBl. I, S.512). Der in der vorliegenden Erfindung verwendete Sirup enthält 10 bis 30%, vorzugsweise 15 bis 30% Wasser.

Bezüglich der Ausführungsformen der Weichmacher, Stärke und Zuschlagsstoffe wird auf die entsprechenden Ausführungen zum Verfahren verwiesen.

Wenn die Masse als Film ausgebildet ist und für die Herstellung von einteiligen Kapselhüllen im Rotary-Die-Prozess herangezogen werden soll, ist eine Bruchdehnung bei Verkapselungstemperatur von 40°C bis 90°C von mindestens 100% erforderlich, bevorzugt liegt die Bruchdehnung jedoch bei mindestens 160% und noch bevorzugter mindestens 240%.

Der Formkörper, insbesondere die aus dem Film gebildete Weichkapselhülle hat bei 25°C und 60% relativer Luftfeuchtigkeit eine Festigkeit von $\sigma_m$, bevorzugt mindestens 3,5 MPa und noch bevorzugter mindestens 5 MPa.

Teil der Erfindung sind weiterhin Formkörper, welche aus der erfindungsgemässen Masse hergestellt werden.

Weiterhin Teil der Erfindung ist eine einteilige Kapselhülle, welche Stärke mit einem Staudinger-Index von mindestens 40 ml/g, bevorzugt von mindestens 50 ml/g und noch bevorzugter von mindestens 80ml/g enthält. Besonders vorteilhaft ist eine Kapsel mit einem Staudinger-Index der Stärke von 100 ml/g und noch besser einen Staudinger-Index von 130ml/g.

Die erfindungsgemässen Massen eignen sich gut für die Herstellung von Mehrkammer- bzw. Zweikammerkapseln wie sie z. B. in WO 00/28976 beschrieben sind. Da der Wassergehalt des Filmes bzw. der Filme gering eingestellt werden kann, treten in den fertigen getrockneten Kapseln, insbesondere in den die Kammern bildenden Trennwänden, nahezu keine Spannungen auf, was die Stabilität der Mehrkammerkapsel im Vergleich zu Mehrkammer-Weichgelatinekapseln wesentlich erhöht.

Beispielsweise lassen sich Zweikammerkapseln realisieren, deren eine Kammer mit Pulver oder Granulat gefüllt ist und deren andere Kammer eine Flüssigkeit enthält

Der Formkörper, insbesondere die Kapselhülle hat eine Dicke im Bereich zwischen 0,1 und 2 mm bevorzugt zwischen 0,2 und 0,6 mm.

In einer weiteren bevorzugten Ausführungsform besteht der Formkörper, insbesondere die Weichkapselhülle aus einem mehrlagigen Film. Mindestens zwei der Filme haben eine unterschiedliche chemische Zusammensetzung.

Abgesehen von der Herstellung einschichtiger Kapselhüllen kann die thermoplastisch verarbeitbare Stärkeschmelze auch zur Herstellung jeglicher anderer Art von Formkörper insbesondere Verpackungsmaterialien verwendet werden. Im thermoplastischen Zustand ist die Masse verarbeitbar, insbesondere extrudierbar.

In einer besonders bevorzugten Ausführungsform wird die Zusammensetzung der erfindungsgemässen Masse so gewählt, dass die Weichmacher, welche in der Masse verbleiben, und der Wassergehalt, der mit der Umgebung in ständigem Austausch steht, dem Herstellungsverfahren derart angepasst sind, dass die Stärkekörner destrukturiert, zu einer homogenen Masse geschmolzen, zu einem Band oder Film extrudiert werden können und dieses Band zur Herstellung von Formkörpern mit Hilfe des Rotary-Die-Verfahrens verwendet werden kann, ohne dass das Endprodukt Kapsel und sämtliche während des Verfahrens entstehenden Zwischenprodukte getrocknet werden müssen.

Das in Schritt a) verwendete Biopolymer kann Stärke oder modifizierte Stärke in nativer oder kristalliner nicht destrukturierter Form mit einem Feuchtigkeitsgehalt von 10 bis 30%, vorzugsweise von 15 bis 23% sein.

Die Schmelztemperatur in Schritt b) beträgt zwischen 70 und 180°C, vorzugsweise zwischen 80 und 160°C. Der in diesem Schritt angewendete Druck entspricht mindestens dem Wasserdampfdruck der jeweiligen Temperatur. Wenn notwendig kann bei diesem Schritt Wasserdampf in einer Dekompressionszone aus der Verarbeitungsvorrichtung abgelassen oder Wasser in einer Einspritzzone in die Verarbeitungsvorrichtung eingeführt werden.

Der in Schritt d) unter einem erhöhten Druck von > 50 at und bei einer Temperatur von 80 bis 105°C gebildete Film weist eine minimale Dicke von 0,2 mm bis zu einer maximalen Dicke von 2 mm auf.

Die in Schritt e) mit Hilfe des Rotary-Die-Verfahrens gebildeten Formkörper können wahlweise mit einer Lösung, Emulsion, Suspension oder einer Trockensubstanz gefüllt werden. Es kann sich hierbei um pharmazeutische Stoffe oder Stoffgemische handeln.

Bei der in Schritt f) beschriebenen Anpassung oder Steuerung werden die Formkörper Standardbedingungen angepasst, d.h. an eine relative Feuchtigkeit von weniger als 50% und an eine Temperatur von weniger als 30°C, wobei ±2 bis vorzugsweise ±5% des absoluten Wassergehalts der Masse ab- oder zugeführt wird.

Eine besonders bevorzugte, erfindungsgemässe Masse besitzt einen Staudinger Index von >50 ml/g und ein E-Modul von weniger als 2 MPa bei 60 bis 90°C und weniger als 20% Wasser.

Bei Umgebungstemperatur (Gebrauchstemperatur: 10-30°C) und im Gleichgewicht mit der relativen Luftfeuchtigkeit (20-75%) der Umgebung sollte der Film einen Staudinger Index von grösser als 40 ml/g, eine minimale Elastizität von 10% und eine minimale Festigkeit von zum Beispiel 5 Mpa aufweisen. Dieser Parameter und auch die Festigkeit (Festigkeitstester Bareiss, (N)) und Klebrigkeit (die Tendenz unter dem Eigengewicht aneinander zu kleben) wird zu einem grossen Teil durch das Gleichgewicht zwischen Wassergehalt und Weichmacher auf der einen Seite und der Luftfeuchtigkeit der Umgebung auf der anderen Seite beeinflusst.

Der Wassergehalt des Weichmachers ist gleichermassen für die Sorption, Festigkeit, das E-Modul und die Schmelzflussgeschwindigkeit bei erhöhten und normalen Temperaturen von Bedeutung. Es ist unabdingbar, dass nicht nur Polyole reiner chemischer Natur (wie Glycerin und Sorbitol) verwendet werden, sondern gerade Polyol-Mischungen (einschliesslich höherer molekularer Formen), die alle aus enzymatischer und/oder chemische Hydrolyse von Stärke gefolgt von einer Hydrierung hergestellt werden. Diese Produkte können in den meisten Fällen nicht als Trockensubstanzen verkauft werden, da aufgrund der unterschiedlichen chemischen Eigenschaften und der Kettenlänge der enthaltenden Moleküle keine Kristallisierung stattfinden kann. Der Wassergehalt des zu verwendenden Sirups (vorzugsweise ein Gemisch aus Sorbitol/Mannitol-Sirup) muss für das gesamte erfindungsgemässe Verfahren mit einberechnet werden.

Ein weiterer wichtiger Faktor zur Kontrolle der Wasseraktivität während der Extrusion und daher für das gesamte, in dieser Erfindung beschriebene Verfahren ist die kontrollierte Abführung des Wasserdampfs. Dies wird durch eine Regulierung des Gesamtdrucks während der Extrusion an verschiedenen Punkten der Temperatur/Druck-Kurve entlang der Achse des Extrusionssystems durch Druckventile und durch Aufrechterhalten eines kontrollierten Drucks (zwischen 0 und -0,95 mbar) oder durch Einbringen sehr kleiner Wassermengen reguliert, um die notwendige Gleichgewichtskonzentration zu erreichen und zu erhalten. Die in der Masse enthaltene absolute Wassermenge sollte im Bereich von ±1% bis ±5%, vorzugsweise jedoch ±2% zu der Gebrauchsfeuchte liegen. Die Gebrauchsfeuchte ist die Feuchtigkeit, die sich in Gebrauchs- und Umgebungsfeuchte von 20 - 75% rel. H. durch Sorption in der Hülle einstellt.

Bei der Doppelschneckenextrusion werden eine oder mehrere sogenannte Entgasungszonen eingesetzt, über die das überschüssige Wasser (aus der Stärke und/oder dem Polyolsirup) der Schmelze entzogen wird. Durch Einstellen des in der Schmelze wirksamen Unterdrucks (z.B. mit Hilfe eines Frischluftventils zwischen Vakuumpumpe und Extruder) kann der Endwassergehalt des extrudierten Materials gezielt eingestellt werden.

Bei festem Gesamtdurchsatz wird zum Beispiel mit einem in den Entgasungszonen wirksamen absoluten Druck von 200 mbar erreicht, dass der Endwassergehalt des Extrudats z.B. 6% beträgt; wird der Druck auf 450 mbar eingestellt, so resultiert ein Endwassergehalt von zum Beispiel 12%.

Ein Ausführungsbeispiel der Erfindung in vorrichtungsmässiger Hinsicht ist in den Figuren dargestellt und wird nachstehend genauer beschrieben.

Es zeigen:

Figur 1      die Bruchdehnung [$\varepsilon_b$] einer Stärke enthaltenden Masse in Abhängigkeit des Staudinger-Index [$\eta$],

Figur 2      eine stark schematisierte Darstellung einer Füll- und Formstation im Rotary-Die-Verfahren,

Figur 3      die symbolische Darstellung eines Doppel-Schneckenextruders mit den darin herrschenden Temperaturverhältnissen,

Figur 4      zeigt das Young'sche Elastizitätsmodul E [MPa] einer erfindungsgemässen Stärke enthaltenden, homogenisierten Masse in Abhängigkeit von der Temperatur (konditioniert 50% relative Luftfeuchtigkeit),

Figur 5      ein Sorbtionsdiagramm Wassergehalt/relative Luftfeuchte zum Beispiel 1, und

Figur 6      ein Sorbtionsdiagramm Wassergehalt/relative Luftfeuchte zum Beispiel 2.

Die Messung der Bruchdehnung und des Young'schen Elastizitätsmoduls E erfolgt nach DIN-Norm 53455 bzw. DIN EN ISO 527-1 bis ISO 527-3. Die Bruchdehnungsmessung erfolgt nach dieser DIN Norm bei der entsprechenden Verkapselungstemperatur.

Die Messung des Staudinger-Index [$\eta$] erfolgt analog der DIN-Norm: DIN 51562-1 bis 51562-4. Allerdings musste nun der Weichmachergehalt der Proben und sein Einfluss auf die Durchlaufzeiten im Ubbelohde-Viskosimeter berücksichtigt werden. Hierzu wurde zuerst der Einfluss des Weichmachergehalts auf die Durchflusszeit $t_0$ bestimmt, mittels der erhaltenen Eichgeraden konnten dann die Durchflusszeiten $t_{0Weichg}$ bei einem beliebigen Weichmachergehalt entsprechend

$$t_{0Weichg} = t_0 \cdot (1,00002 + 0,00238 \cdot c_{Weichg})$$

berechnet werden, wobei $c_{Weichg}$ die vorliegende Konzentration Weichmacher in mg/ml ist. Die für die abgebauten Stärken bestimmten Staudinger-Index sind zusammen mit den mechanischen Eigenschaften der zugehörigen Proben in der Tabelle 1 aufgeführt.

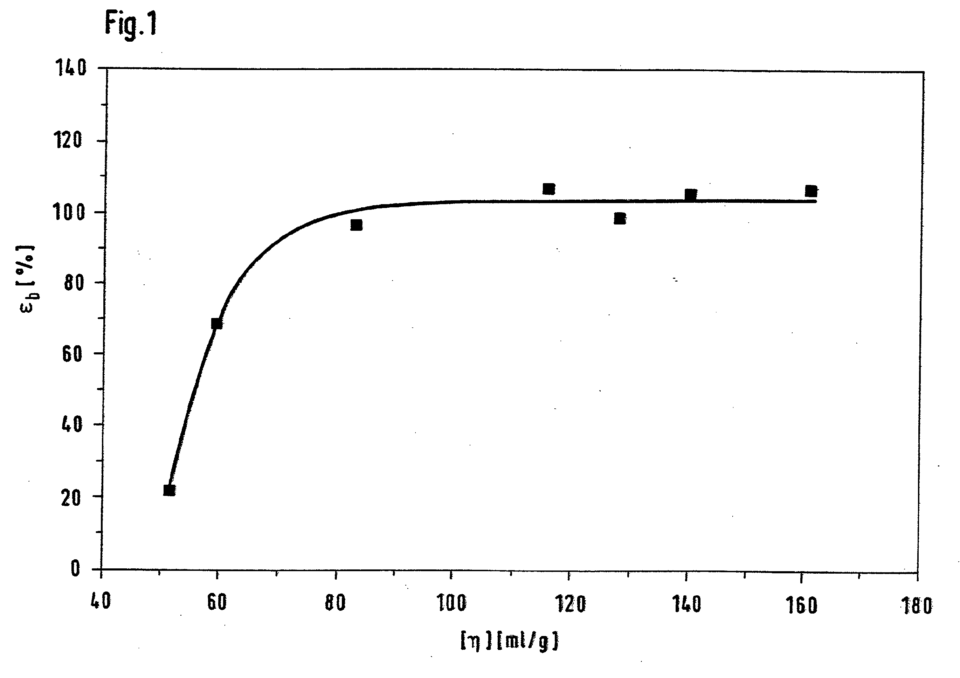

Die Herstellung der Proben, welche in Figur 1 den Zusammenhang zwischen Bruchdehnung und Staudinger-Index demonstrieren erfolgt folgendermassen:

Stärke        56,2 bis 56,9 Gew.%
Glycerin:     41,8 Gew.% bezogen auf den Gehalt der wasserfreien Stärke
Wasser:      1,3 - 2,0 Gew.% bezogen auf das Gesamtgew. der Mischung

Die Mischungen wurden im Brabender-Kneter bei 160rpm und einer Knetzeit von jeweils 15 min bei variablen Knettemperaturen von 110°C, 160°C, 200°C, 220°C und 235°C homogenisiert.

Figur 1 zeigt die Abhängigkeit der Bruchdehnung der Stärke enthaltenden Masse vom Staudinger-Index der Stärke. Aus Figur 1 und dazugehöriger Tabelle 1 ist ersichtlich, dass mit zunehmender Temperatur im Brabender-Kneter der Staudinger-Index der Stärke abnimmt, d.h. bei ansonsten unveränderter Zusammensetzung und unveränderten Prozessparametern (einzige Variable ist die Temperatur) nimmt der Abbaugrad der Stärke zu. 97% Bruchdehnung wird bei einem Staudinger-Index von 82,8 ml/g erreicht. Danach läuft die Bruchdehnung mit zunehmendem Wert des Staudinger-Index asymptotisch einem Grenzwert von ca. 105% zu.

Der Anfangswert des Staudinger-Index, d.h. der Wert ab dem ein merklicher Anstieg der Bruchdehnung beobachtet wird, ist unabhängig vom Weichmacheranteil und einzig abhängig vom Molekulargewichtsmittel der Stärkemoleküle bzw. den entsprechenden Staudinger-Index. Bei geringerem Anteil des Weichmacheranteils verläuft die Kurve insgesamt flacher, d.h. zu tieferen Bruchdehnungen verschoben.

Auch wenn der Staudinger-Index der Gesamtmasse gemessen wird, ist der Wert des Index im wesentlichen nur abhängig vom Polymerisationsgrad der Stärke. Der Wert des Staudinger-Index ist im wesentlichen unabhängig von den übrigen Komponenten der Masse (bzw. ihr geringer Einfluss kann rechnerisch berücksichtigt werden).

Die maximale Festigkeit $\sigma_m$ wurde analog DIN-Norm 53455 bzw. DIN EN ISO 527-1 bis ISO 527-3 bestimmt. Auch $\sigma_m$ zeigt eine Abhängigkeit vom Staudinger-Index, d.h. Abbaugrad der Stärke. Je geringer der Staudinger-Index bei ansonsten unveränderten Bedingungen, desto geriner $\sigma_m$.

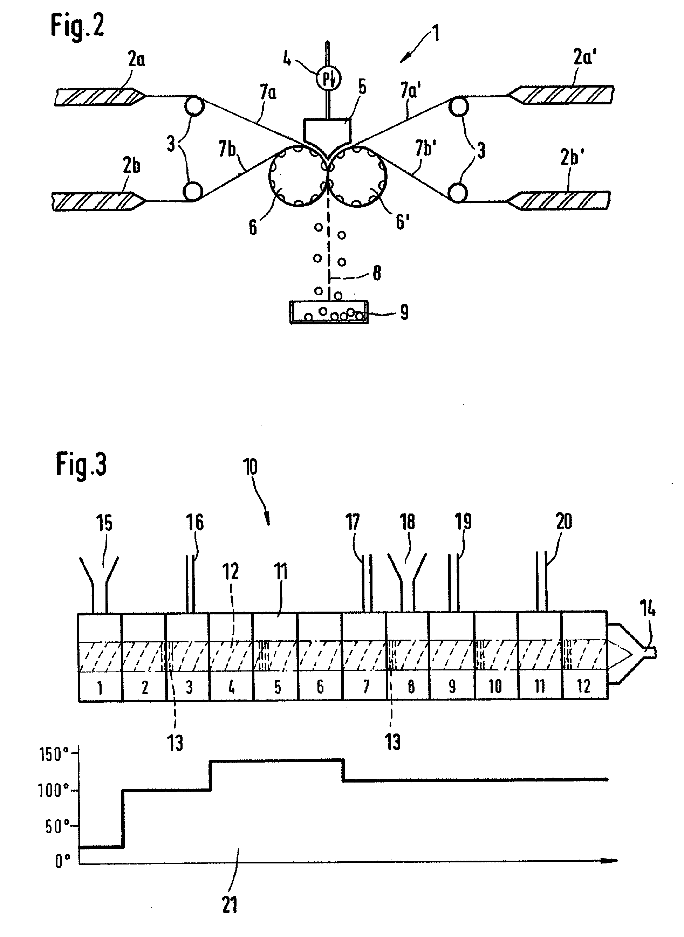

Die insgesamt mit 1 bezeichnete Füll- und Formstation in Figur 2 weist für die Verkapselung auf an sich bekannte Weise ein Formwalzenpaar 6, 6' auf, wobei in den Oberflächen der Formwalzen, die zur Formung der Kapseln erforderlichen Ausnehmungen angeordnet sind. Im Einzugszwickel des Formwalzenpaars ist ein Füllkeil 5 angeordnet, durch den mittels einer Förderpumpe 4 das Füllgut eingebracht werden kann. Beim vorliegenden Ausführungsbeispiel besteht die Kapselhülle aus zwei Schichten mit verschiedenen Materialeigenschaften, welche durch die beiden Stärkefilme 7a, 7a' einerseits und 7b, 7b' andererseits gebildet werden. Diese beiden Stärkefilme werden in den Schneckenextrudern 2a, 2a' und 2b, 2b' aufbereitet und über Umlenkwalzen 3 unmittelbar und mit gleicher Fördergeschwindigkeit dem Einzugszwickel des Formwalzenpaars 6, 6' zugeführt. Die Schneckenextruder sind dabei unmittelbar neben der Füll- und Formstation und gegebenenfalls auf dem gleichen Maschinengestell angeordnet.

Die Stärkefilme werden zwischen dem Formwalzenpaar zu einer einteiligen Weichkapsel geformt und verschweisst, wobei sie das Füllgut einschliessen. Die einzelnen Kapseln 9 werden aufgefangen und allenfalls einem Trocknungsprozess zugeführt, während das verbleibende Filmskelett 8 evtl. durch Recycling wieder zu neuen Kapseln verarbeitet werden kann.

Die unmittelbare Anordnung des Extruders neben der Form- und Füllstation und die "inline" Zuführung des extrudierenden Films in die Form- und Füllstation (ohne Zwischenlagerung) ist natürlich jederzeit möglich, also auch beim Herstellen einschichtiger Kapselhüllen (gängiges Rotary-Die-Verfahren).

Figur 3 zeigt stark vereinfacht einen Doppelschneckenextruder 10, der im vorliegenden Fall aus zwölf einzelnen Gehäuseblöcken 1 - 12 zusammengesetzt ist. Die Gehäuseblöcke sind von links nach rechts durchgehend numeriert. Jeder Gehäuseblock lässt sich mit einem separaten Regelkreis elektrisch heizen und/oder mit ventilgesteuerten Zuflüssen mit Kaltwasser kühlen. Ausserdem können einzelne Blöcke mit Anschlussstutzen versehen sein, wie nachstehend noch erläutert wird. Im vorliegenden Fall handelt es sich um einen gleichdrehenden, engkämmenden Doppelschneckenextruder, wobei der Durchmesser einer Schnecke 44 mm beträgt. Die Länge der gesamten Schneckenwelle beträgt 2'112 mm, was einem Verhältnis von Länge zu Durchmesser von 48 entspricht. Am Ende des Extruders wird das Material über eine Düse 14 ausgetragen. Diese Düse kann beispielsweise zwölf Lochbohrungen von 2 mm Durchmesser aufweisen. Es wäre dabei denkbar, für die Granulatherstellung die einzelnen Materialstränge heiss abzuschlagen und dann einem Fluidbetttrockner zuzuführen. An der Düse 14 könnte aber auch unmittelbar ein fertiger Materialfilm abgezogen werden. Für die Granulatherstellung als Zwischenprodukt kann auch ein Einwellenextruder eingesetzt werden.

An den Schnecken 12 sind an geeigneten Stellen Knetscheiben 13 von unterschiedlicher Konfiguration angeordnet, um eine möglichst homogene Knetung der Materialmischung zu erreichen. Der Block 1 ist wassergekühlt und mit einem Pulvereinzug 15 versehen. Der Block 2 ist geschlossen während am Block 3 eine Einspritzdüse 16 für eine Flüssigdosierung in den Knetraum angeordnet ist. Im Übergangsbereich der Blöcke 2 und 3 sind feine neutrale Knetscheiben 13 angeordnet. Die Blöcke 4 bis 6 sind wiederum geschlossen, wobei an Block 5 breite, neutrale und rückfördernde Knetscheiben vorgesehen sind. Block 7 verfügt über eine Anschlussleitung 17, die mit einer Unterdruckquelle verbunden ist. An Block 8 ist wiederum ein Pulvereinzug 18 angeordnet und die Schnecke mit feinen, neutralen und oder fördernden Knetscheiben versehen. Block 9 verfügt ebenfalls über eine Einspritzdüse 19, während Block 10 geschlossen ist. Die Schnecke im Block 10 verfügt dagegen über breite, neutrale und rückfördernde Knetscheiben. Block 11 hat eine weitere Abzugleitung 20, die mit einer Unterdruckquelle oder mit der Atmosphäre verbunden sein kann. Block 12 ist geschlossen, die Schnecke dort verfügt jedoch über mittlere, fördernde Knetscheiben.

Unterhalb der schematischen Förderschnecke ist eine Temperaturkurve aufgezeichnet. Die einstellbare Temperaturgenauigkeit beträgt +/- 3°C. Bei den angegebenen Temperaturen handelt es sich um die Blocktemperatur, die nicht zwingend mit der Temperatur in der Schmelze identisch sein muss. Die Temperatur in der Schmelze wird ersichtlicherweise noch durch andere Parameter beeinflusst, insbesondere durch die Drehzahl der Schnecke. Bei der Extrusion ist es deshalb erforderlich, diesen Gegebenheiten Rechnung zu tragen und die verstellbaren Grössen derart aufeinander abzustimmen, dass optimale Materialeigenschaften erzielt werden.

Bei dem anhand dieser Figur beschriebenen Ausführungsbeispiel wird eine Drehzahl von 340 Umdrehungen pro Minute (rpm) gefahren. Der gesamte Durchsatz beträgt ca. 34,3 kg/h und die Energieaufnahme beträgt ca. 0,175 kWh/kg. An dem auf 20°C gehaltenen Block 1 wird 20 kg/h (ca. 60%) Stärkepulver zudosiert. Das Pulver wird mit Schubkanten eingezogen und den auf 100°C geheizten Blöcken 2 und 3 zugeführt. Bei Block 3 erfolgt eine Zudosierung von 11 kg/h (ca. 30%) Polyole, zum Beispiel Glycerin mit einem Arbeitsdruck von mindestens 10 bar über eine gravimetrische Kolbenpumpe. In den geschlossenen Blöcken 4 bis 6 ist die Temperatur bis auf 140°C erhöht. Bei Block 7 ist ein Unterdruck von 800 mbar angelegt, wobei ca. 6% Wasser abgehen. Die Temperatur ist jetzt wieder auf 110°C zurückgenommen. Bei Block 8 erfolgt eine Zufuhr von 1,4 kg/h (ca. 10%) Kalziumcarbonat. Gegebenenfalls kann an Block 9 1,9 kg/h (ca. 5 bis 8%) Polyole, zum Beispiel Glycerin zudosiert werden. Der Arbeitsdruck beträgt ebenfalls mindestens 10 bar. Falls dieser Anschluss nicht benötigt wird, ist er mit einem Blindstopfen verschlossen. Bei Block 11 ist wiederum ein Unterdruck angelegt, wobei ca. 2 bis 4% Wasser abgehen. Gegebenenfalls genügt aber auch eine nur atmosphärische Belüftung.

Die Temperatur der Schmelze darf an keiner Stelle des Extruders 160°C überschreiten, weil sonst ein thermischer Abbau der Stärke einsetzt. Weiter gilt, dass die thermische Veränderung der Stärke um so geringer ausfällt, je kürzer die Schmelze einer hohen Temperatur ausgesetzt wird. Zwischen Temperatursteuerung und Materialdurchsatz muss daher ein optimales Verhältnis hergestellt werden.

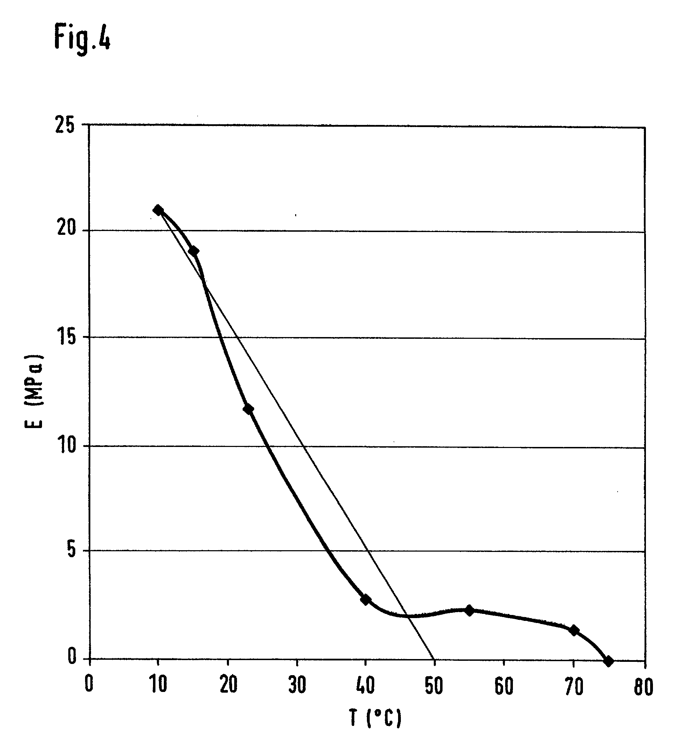

In Figur 4 ist die Temperaturabhängigkeit des Young'schen Elastizitätsmoduls E illustriert. Die Zusammensetzung der Prüflinge entspricht Beispiel 2 (durchgezogene Linie). Im Vergleich dazu ist das theoretische Temperaturverhalten eines Thermoplasten ähnlicher Glasübergangstemperatur dargestellt. Während das E-Modul des "normalen" Thermoplasten (gestrichelte Linie) rasch linear auf Null abfällt, ist bei den Prüflingen das E-Modul in einem Bereich von 40°C bis ca. 70°C nahezu unabhängig von der Temperatur. Dieses Verhalten ist unter anderem auch verantwortlich für die vorteilhaften Eigenschaften der vorliegenden Erfindung.

Die vorliegende Erfindung wird anhand der nachstehenden Beispiele weiter erläutert:

**Beispiel 1:**

Über einen Zweiwellenextruder (Typ ZSK 25, Krupp, Werner & Pfleiderer) werden folgende Komponenten kontinuierlich dosiert und in den thermoplastisch verarbeitbaren Zustand überführt:
Alle Angaben in Gewichtsteilen:

| Zusammensetzung zu Beginn des Verfahrens: Hydroxypropylierte Kartoffelstärke, native granuläre Form | |
|---|---|
| 21% Feuchte (abs. Wassergehalt) | |
| 0,1 Mol% Hydroxypropylgruppen | 60,2 Teile |
| Sorbitolsirup FCCIII (70% Trockenmasse) | 37,5 Teile |
| Flüssiges Lecithin FCIII | 1,1 Teile |
| Glycerin Monostearat | 1,2 Teile |

Gesamte Wassermenge zu Beginn der Extrusion:23,8%

Zusammensetzung am Verfahrensende im Gleichgewicht mit der Umgebungsluftfeuchtigkeit: 25% C, 60% relH
Wassergehalt gemessen mit Karl Fischer: 11,5%

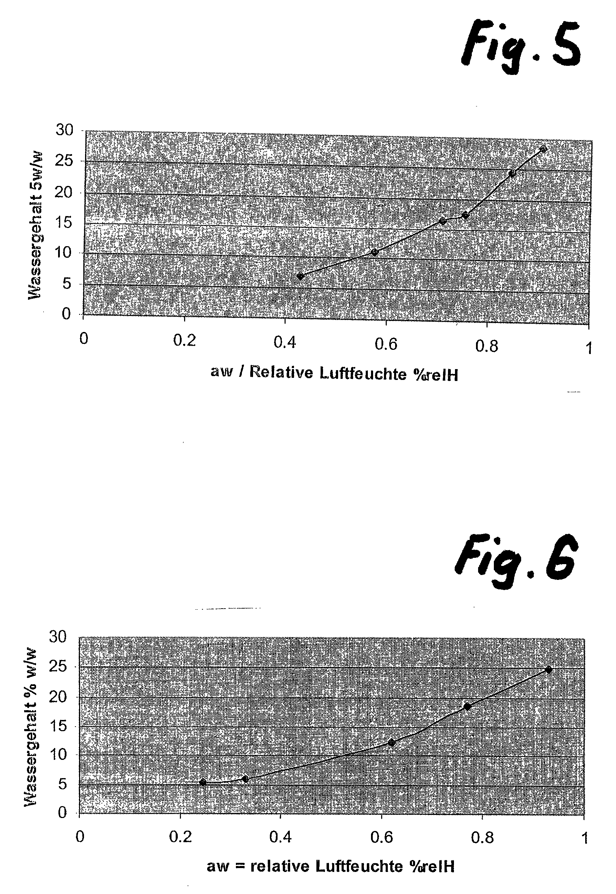

Sorptions-Diagramm siehe Figur 5

**Beispiel 2:**

Über einen Zweiwellenextruder (Typ ZSK 25, Krupp, Werner & Pfleiderer) werden folgende Komponenten kontinuierlich dosiert und in den thermoplastisch verarbeitbaren Zustand überführt:
Alle Angaben in Gewichtsteilen:

| Native Kartoffelstärke; | |
|---|---|
| 20,1% Feuchte (abs. Wassergehalt) | 61,0 Teile |
| Maltitolsirup FCCIII (74,5% Trockenmaterial) | 13,0 Teile |
| Sorbitolsirup FCIII (70% Trockenmaterial) | 21,0 Teile |
| Glycerin Monostearat (40% Mono-Ester) E471 | 1,0 Teile |

(fortgesetzt)

| Native Kartoffelstärke; | |
|---|---|
| Glycerin 98% USP | 4,0 Teile |

Gesamte Wassermenge zu Beginn der Extrusion: 28.3 % Zusammensetzung nach der Extrusion und im Gleichgewicht mit der Umgebungsluftfeuchtigkeit: 25 % C, 50 % rel H: Wassergehalt 10,5 %

Sorptions-Diagramm siehe Figur 6

Tab. 1:

| Die mechanischen Eigenschaften der Stärkefilme mit 41,8% Glycerin in Abhängigkeit des Staudinger-Index [η] | | | | | |
|---|---|---|---|---|---|
| $T_B$ °C | $H_2O$ % | [η} ml/g | d mm | $\sigma_m$ MPa | $\varepsilon_b$ % |
| 110 | 1.77 | 160.5 | 0.72 | 7.0 +/- 0.3 | 107 +/- 6 |
| 140 | 1.80 | 139.9 | 0.65 | 6.8 +/- 0.4 | 106 +/- 18 |
| 160 | 1.55 | 127.9 | 0.64 | 6.3 +/- 0.4 | 99 +/- 5 |
| 180 | 1.54 | 115.6 | 0.64 | 6.9 +/- 0.2 | 107 +/- 9 |
| 220 | 1.66 | 82.8 | 0.73 | 4.8 +/- 0.4 | 97 +/- 23 |
| 200 | 1.55 | 59.2 | 0.61 | 4.9 +/- 0.5 | 69 +/- 23 |
| 235 | 1.30 | 51.5 | 0.87 | 9.0 +/- 0.7 | 22 +/- 24 |

**Patentansprüche**

1. Verfahren zum Herstellen von Formkörpern, insbesondere von Weichkapseln, **gekennzeichnet durch** folgende Schritte:

   a) Mischen wenigstens eines ersten pflanzlichen Biopolymers in Pulver- oder Granulatform mit wenigstens einem Weichmacher in flüssiger Form, insbesondere in Form eines Sirups, gegebenenfalls zusammen mit Zuschlagstoffen, zu einem homogenen Rohstoffgemisch;
   b) Aufschmelzen des Rohstoffgemischs unter Zufuhr von Wärme und unter gegenüber der Athmosphäre erhöhtem Druck in einer Verarbeitungseinrichtung, insbesondere in einer Extrusionsstufe, zu einer thermoplastisch verarbeitbaren Masse;
   c) gegebenenfalls Herstellen eines Zwischenprodukts, insbesondere eines Granulats, nach Abkühlen der in Schritt b) erhaltenen Masse, und erneute Aufbereitung zu einer thermoplastisch verarbeitbaren Masse;
   d) Ausbilden wenigstens eines Films aus der thermoplastisch verarbeitbaren Masse gemäss Schritt b) oder gegebenenfalls Schritt c), insbesondere **durch** Extrusion aus einer Schlitzdüse;
   e) Herstellen von Formkörpern unter Verwendung des Films in einem intermittierenden oder kontinuierlichen Verfahren an einer Formstation, insbesondere an einer Rotary-Die-Verkapselungsmaschine;
   f) wobei über sämtliche Prozessstufen vom Rohstoffgemisch bis zu den fertigen Formkörpern der Feuchtigkeitsgehalt aller Komponenten derart gewählt bzw. gesteuert wird, dass die Restfeuchte der fertigen Formkörper nach dem Verlassen der Formstation unter Vermeidung eines Trocknungsprozesses dem gewünschten Wert bei Lager- bzw. Gebrauchsbedingungen entspricht.

2. Verfahren nach Anspruch 1, **dadurch gekennzeichnet, dass** das erste pflanzliche Biopolymer Stärke oder modifizierte Stärke in nativer bzw. kristalliner, nicht destrukturierter Form ist.

3. Verfahren nach Anspruch 1 oder 2, **dadurch gekennzeichnet, dass** das Rohstoffgemisch wenigstens ein zweites Biopolymer enthält, das ausgwählt ist aus einer Gruppe bestehend aus Stärke, modifizierte Stärke, Zellulose, insbesondere teilhydroxypropylierte Cellulose, Alginate, Pektine, Agar, Carrageenan (lambda-, iota- oder kappa-Carrageenan), Galactomannane (Guar- und Johannisbrotkernmehl), Xanthan-Gummi, Tamarinde, Tragant-Gummi, Karaya-Gummi, Chitosan, Glucomannane, Casein, Dextrine, Cyklodextrine, Pullulan und Arabinogalaktan.

**4.** Verfahren nach einem der Ansprüche 1 bis 3, **dadurch gekennzeichnet, dass** das erste und gegebenenfalls das zweite pflanzliche Biopolymer einen Feuchtigkeitsgehalt von 10 % bis 30 %, vorzugsweise von 15 % bis 23 % hat.

**5.** Verfahren nach einem der Ansprüche 1 bis 4, **dadurch gekennzeichnet, dass** das Rohstoffgemisch einen Weichmacher enthält, der ausgewählt ist aus einer Gruppe bestehend aus Glycerin, Sirup aus hydrierter, teilhydrolisierter Stärke, Abbauprodukten enthaltend Oligosaccharide und Monosaccharide, Sorbitol, Maltitol, Mannitol, Erythritol, Xylitol, Spuren reduzierender Zucker und Gemische davon.

**6.** Verfahren nach einem der Ansprüche 1 bis 5, **dadurch gekennzeichnet, dass** der Weichmacher in der Form eines Polyolsirups einen Wassergehalt von 15 % bis 30 % aufweist.

**7.** Verfahren nach einem der Ansprüche 1 bis 6, **dadurch gekennzeichnet, dass** wenigstens 1 Gewichtsprozent mehr eines von hydrolysierter und hydrierter Stärke stammenden Polyols als von anderen Weichmachern vorhanden ist.

**8.** Verfahren nach einem der Ansprüche 1 bis 7, **dadurch gekennzeichnet, dass** das Aufschmelzen des Rohstoffgemisches vorzugsweise bei einer Temperatur von 80° bis 160° Celsius erfolgt und dass der Druck wenigstens dem Dampfdruck bei dieser Temperatur entspricht, wobei Wasserdampf in einer Dekompressionszone aus der Verarbeitungsvorrichtung abgelassen oder Wasser in einer Einspritzzone in die Verarbeitungsvorrichtung eingespritzt wird.

**9.** Verfahren nach einem der Ansprüche 1 bis 8, **dadurch gekennzeichnet, dass** die Ausbildung des Films durch Extrusion unter einem Druck von über 50 at und unter einer Temperatur von 80° bis 105° Celsius aus einer Schlitzdüse in eine atmosphärische Umgebung erfolgt.

**10.** Verfahren nach einem der Ansprüche 1 bis 9, **dadurch gekennzeichnet, dass** ein Film in einer Schichtdicke von 0,2 mm bis 2 mm ausgebildet wird.

**11.** Verfahren nach einem der Ansprüche 1 bis 10, **dadurch gekennzeichnet, dass** der Film einen staudinger Index von wenigstens 40 ml/g, vorzugsweise wenigstens 50 ml/g und noch bevorzugter wenigstens 80 ml/g aufweist.

**12.** Verfahren nach einem der Ansprüche 1 bis 11, **dadurch gekennzeichnet, dass** die Oberfläche der Formkörper zur Reduzierung einer Feuchtigkeitsaufnahme oder eines Feuchtigkeitsverlusts mit einer lipophilen oder wachshaltigen Versiegelungssubstanz beschichtet wird.

**13.** Verfahren nach Anspruch 12, **dadurch gekennzeichnet, dass** die Versiegelungssubstanz ausgewählt ist aus einer Gruppe bestehend aus Bienenwachs (E901), Karnaubawachs (E903), Candellilawachs (E902), Beeren-Wachs, Montanglycol-Wachs (E912), Polyethylenglykol-Wachsoxidate (E914), Shellac (E904), Mono-, Di- und Triglyceride von Speisefettsäuren (E471, Zuckerester von Speisefettsäuren (E476) und Dimetzylpolysiloxan (E900).

**14.** Formkörper, insbesondere Weichkapseln, hergestellt nach einem der Ansprüche 1 bis 13.

Fig.1

EP 1 258 242 A1

## Fig.2

## Fig.3

Fig.4

## Fig. 5

Y-axis: Wassergehalt 5w/w (0, 5, 10, 15, 20, 25, 30)
X-axis: aw / Relative Luftfeuchte %relH (0, 0.2, 0.4, 0.6, 0.8, 1)

## Fig. 6

Y-axis: Wassergehalt % w/w (0, 5, 10, 15, 20, 25, 30)
X-axis: aw = relative Luftfeuchte %relH (0, 0.2, 0.4, 0.6, 0.8, 1)

Europäisches
Patentamt

**EUROPÄISCHER RECHERCHENBERICHT**

Nummer der Anmeldung

EP 01 11 1739

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| E | EP 1 103 254 A (GREITHER PETER) 30. Mai 2001 (2001-05-30) * Absatz '0063!; Ansprüche 1-6,9,10,17,20 * | 1-5,8, 10,11,14 | A61K9/48 |
| X,D | WO 90 14938 A (GOODMAN FIELDER WATTIE AUSTRALIA LTD (AU)) 13. Dezember 1990 (1990-12-13) * Seite 11, Zeile 17 - Seite 17, Zeile 21 * | 1-5,8, 11,14 | |
| X,D | US 4 738 724 A (WITTWER FRITZ ET AL) 19. April 1988 (1988-04-19) * Spalte 7, Zeile 36 - Spalte 9, Zeile 2 * * Spalte 22, Zeile 4-8 * * Spalte 26, Zeile 54-59 * * Spalte 30, Zeile 32-42 * * Ansprüche 1,3-6 * | 1-5,8,14 | |
| Y | | 12,13 | |
| Y | US 4 350 679 A (MIZUNO YASUHIKO ET AL) 21. September 1982 (1982-09-21) * Spalte 1, Zeile 14-33 * * Spalte 1, Zeile 47-53 * | 12,13 | RECHERCHIERTE SACHGEBIETE (Int.Cl.7) A61K A61J |
| X | DE 37 12 029 A (WARNER LAMBERT CO) 12. November 1987 (1987-11-12) * Seite 3, Zeile 22 - Seite 5, Spalte 42; Beispiel 1 * | 1-5,8,14 | |
| A,D | WO 97 35537 A (BROWN MALCOLM DAVID ;BIOPROGRESS TECHNOLOGY LIMITED (GB)) 2. Oktober 1997 (1997-10-02) * das ganze Dokument * | 1 | |

Der vorliegende Recherchenbericht wurde für alle Patentansprüche erstellt

| Recherchenort | Abschlußdatum der Recherche | Prüfer |
|---|---|---|
| MÜNCHEN | 23. Oktober 2001 | Rosenblatt, T |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**   EP 01 11 1739

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-10-2001

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| EP | 1103254 | A | 30-05-2001 | EP | 1103254 | A1 | 30-05-2001 |
| | | | | AU | 1262801 | A | 04-06-2001 |
| | | | | WO | 0137817 | A1 | 31-05-2001 |
| WO | 9014938 | A | 13-12-1990 | AU | 627589 | B2 | 27-08-1992 |
| | | | | AU | 5736590 | A | 07-01-1991 |
| | | | | WO | 9014938 | A1 | 13-12-1990 |
| | | | | AT | 126477 | T | 15-09-1995 |
| | | | | CA | 2054202 | A1 | 02-12-1990 |
| | | | | DE | 69021728 | D1 | 21-09-1995 |
| | | | | DE | 69021728 | T2 | 18-01-1996 |
| | | | | DK | 474705 | T3 | 27-12-1995 |
| | | | | EP | 0474705 | A1 | 18-03-1992 |
| | | | | KR | 168680 | B1 | 20-03-1999 |
| | | | | KR | 184845 | B1 | 15-05-1999 |
| | | | | NO | 179616 | B | 05-08-1996 |
| | | | | US | 5314754 | A | 24-05-1994 |
| US | 4738724 | A | 19-04-1988 | BE | 900950 | A1 | 30-04-1985 |
| | | | | CH | 661878 | A5 | 31-08-1987 |
| | | | | DE | 3438656 | A1 | 23-05-1985 |
| | | | | FR | 2554418 | A1 | 10-05-1985 |
| | | | | GB | 2148841 | A ,B | 05-06-1985 |
| | | | | IT | 1178193 | B | 09-09-1987 |
| | | | | JP | 1947556 | C | 10-07-1995 |
| | | | | JP | 6073539 | B | 21-09-1994 |
| | | | | JP | 60132562 | A | 15-07-1985 |
| | | | | AT | 44975 | T | 15-08-1989 |
| | | | | AU | 572119 | B2 | 05-05-1988 |
| | | | | BG | 46154 | A3 | 16-10-1989 |
| | | | | DD | 218113 | A5 | 30-01-1985 |
| | | | | DE | 3479129 | D1 | 31-08-1989 |
| | | | | DK | 76584 | A | 19-08-1984 |
| | | | | EP | 0118240 | A2 | 12-09-1984 |
| | | | | FI | 840614 | A ,B, | 19-08-1984 |
| | | | | IE | 56888 | B1 | 15-01-1992 |
| | | | | IL | 70980 | A | 31-03-1987 |
| | | | | MX | 170518 | B | 27-08-1993 |
| | | | | NO | 840598 | A ,B, | 20-08-1984 |
| | | | | NZ | 207188 | A | 10-09-1986 |
| | | | | PT | 78123 | A ,B | 01-03-1984 |
| | | | | TR | 23047 | A | 14-02-1989 |
| | | | | US | 4673438 | A | 16-06-1987 |
| | | | | CH | 664938 | A5 | 15-04-1988 |
| | | | | DE | 3438235 | A1 | 30-05-1985 |
| | | | | FR | 2555972 | A1 | 07-06-1985 |

EPO FORM P0461

Für nähere Einzelheiten zu diesem Anhang : siehe Amtsblatt des Europäischen Patentamts, Nr.12/82

**EP 1 258 242 A1**

**ANHANG ZUM EUROPÄISCHEN RECHERCHENBERICHT
ÜBER DIE EUROPÄISCHE PATENTANMELDUNG NR.**   EP 01 11 1739

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-10-2001

| Im Recherchenbericht angeführtes Patentdokument | | Datum der Veröffentlichung | Mitglied(er) der Patentfamilie | | Datum der Veröffentlichung |
|---|---|---|---|---|---|
| US 4738724 | A | | GB | 2148235 A ,B | 30-05-1985 |
| | | | IT | 1178161 B | 09-09-1987 |
| | | | JP | 5061942 B | 07-09-1993 |
| | | | US | 4738817 A | 19-04-1988 |
| US 4350679 | A | 21-09-1982 | JP | 1508887 C | 26-07-1989 |
| | | | JP | 56156212 A | 02-12-1981 |
| | | | JP | 63054685 B | 28-10-1988 |
| | | | AT | 4372 T | 15-08-1983 |
| | | | CA | 1179904 A1 | 25-12-1984 |
| | | | DE | 3160721 D1 | 08-09-1983 |
| | | | EP | 0039879 A1 | 18-11-1981 |
| | | | ES | 501992 D0 | 16-08-1982 |
| | | | ES | 8206182 A1 | 16-11-1982 |
| | | | KR | 8401509 B1 | 29-09-1984 |
| | | | PH | 16668 A | 13-12-1983 |
| DE 3712029 | A | 12-11-1987 | CH | 669201 A5 | 28-02-1989 |
| | | | BE | 1000461 A3 | 13-12-1988 |
| | | | DE | 3712029 A1 | 12-11-1987 |
| | | | FR | 2598148 A1 | 06-11-1987 |
| | | | GB | 2190093 A ,B | 11-11-1987 |
| | | | IT | 1205824 B | 31-03-1989 |
| | | | JP | 1974582 C | 27-09-1995 |
| | | | JP | 7000711 B | 11-01-1995 |
| | | | JP | 63010644 A | 18-01-1988 |
| WO 9735537 | A | 02-10-1997 | AU | 726280 B2 | 02-11-2000 |
| | | | AU | 2168597 A | 17-10-1997 |
| | | | BR | 9708352 A | 04-01-2000 |
| | | | CA | 2250397 A1 | 02-10-1997 |
| | | | CZ | 9803079 A3 | 17-02-1999 |
| | | | EP | 0889710 A1 | 13-01-1999 |
| | | | WO | 9735537 A1 | 02-10-1997 |
| | | | JP | 2000515397 T | 21-11-2000 |
| | | | NO | 984472 A | 28-09-1998 |
| | | | TR | 9801923 T2 | 21-08-2000 |

EPO FORM P0461

Für nähere Einzelheiten zu diesem Anhang : siehe Amtsblatt des Europäischen Patentamts, Nr.12/82